(19) Europäisches Patentamt
European Patent Office
Office européen des brevets

(11) EP 0 844 235 B1

(12) # EUROPEAN PATENT SPECIFICATION

(45) Date of publication and mention of the grant of the patent:
**12.09.2001 Bulletin 2001/37**

(51) Int Cl.⁷: **C07C 231/02**, C07C 233/47

(21) Application number: **96118530.3**

(22) Date of filing: **20.11.1996**

(54) **Process for preparation of N-acyl-aminodiacids**

Verfahren zur Herstellung von N-Acylaminodicarbonsäuren

Procédé de préparation des acides dicarboxyliques N-acyl

(84) Designated Contracting States:
**CH DE ES FR GB IT LI**

(43) Date of publication of application:
**27.05.1998 Bulletin 1998/22**

(73) Proprietor: **Clariant Finance (BVI) Limited**
**Road Town, Tortola (VG)**

(72) Inventors:
- **Brown, David M.**
  **Charlotte, NC 28294 (US)**
- **Khadim, Mohammad A.**
  **Huntsville, NC 28078 (US)**
- **Sadanani, Narayan D.**
  **Charlotte, NC 28210 (US)**
- **Yeager, April**
  **Stanley, NC 28164 (US)**

(74) Representative: **Brundin, Eike, Dr. et al**
**Clariant GmbH**
**Patente, Marken, Lizenzen**
**Am Unisys-Park 1**
**65843 Sulzbach (DE)**

(56) References cited:
**DE-A- 2 015 075**

- **DATABASE WPI Section Ch, Week 9204 Derwent Publications Ltd., London, GB; Class D22, AN 92-030641 XP002028871 & JP 03 279 354 A (NISSEI KAGAKU KOGYO KK) , 10 December 1991**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 844 235 B1

Printed by Jouve, 75001 PARIS (FR)

## Description

This invention relates to an improved process for the preparation of N-acyl-aminodiacids.

### Background of the Invention

A variety of methods have been used to prepare N-acyl-aminodiacids. These include the method described in German Auslegeschrift 20 15 075 to Ajinomoto Co., Inc. There still remains a need, however, for an improved process and, in particular, a process that utilizes non-biohazard solvents and that results in improved yields.

### Summary of the Invention

This invention comprises an improved process of making N-acyl-aminodiacids of Formula I:

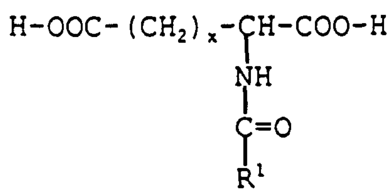

Formula I

wherein:

- $R^1$ is selected from the group consisting of $C_1$ - $C_{20}$ alkyl, $C_2$ - $C_{20}$ alkenyl, $C_2$ - $C_{20}$ alkynyl, wherein the alkenyl and alkynyl groups may contain up to three unsaturations and wherein all of the groups may optionally contain branching; and
- x = 0 - 10, particularly 1 - 4.

A particular compound is lauroyl glutamic acid (LGA) where
$R^1 = C_{11}$ (n-undecyl group) and x = 2.
In the process of the invention an excess of a compound of Formula II:

$$M^1\text{-OOC-CH-}(NH_2)\text{-}(CH_2)_x\text{-COOM}^2 \qquad \text{Formula II}$$

wherein $M^1$ and $M^2$ are each independently selected from the group consisting of hydrogen, sodium, potassium and ammonium, is reacted with a chloride of Formula III:

$$R^1\text{-C(O)Cl} \qquad \text{Formula III}$$

The reaction in conducted in the presence of a water/alcohol mixture with a sufficient amount of base selected from the group consisting of NaOH, KOH, $Na_2CO_3$ and $K_2CO_3$ (particularly caustic) to form a disalt form of the compound of Formula I while maintaining a certain pH value, temperature and molar equivalent of compound III. The disalt (such as the disodium salt) is then added to mineral acid solution to form the compound of Formula I.

### Detailed Description of the Invention

This invention relates to an improved method of making compounds of Formula I. First, a compound of Formula II (for example, monosodium glutamate where one of $M^1$ and $M^2$ is sodium, the other of $M^1$ and $M^2$ hydrogen, and x = 2) is dissolved in water in a clean vessel equipped with a thermometer, overhead stirrer, pH probe, pressure equalizing funnel containing caustic (for example a 50% solution by weight), pressure equalizing funnel with the acyl chloride selected for the reaction and a subsurface addition tube for the sparging of an inert gas (such as nitrogen). For safety reasons and as needed, the atmosphere of the vessel is then treated to create an atmosphere which is free of oxygen

and which will not support combustion. This is done by periodically applying vacuum and purging the vessel with an inert gas such as nitrogen as needed to keep the atmosphere in the vessel in a state where it will not support combustion of an explosive oxygen/solvent mixture in the head space of the vessel.

Next, the vessel is charged with a polar organic solvent selected from the group consisting of methanol, ethanol, n-propanol, isopropanol, n-butanol, isobutanol, tert-butanol and tetrahydrofuran (THF). Preferred solvents are ethanol, n-propanol and isopropanol. Standard precautions should be taken with the reaction solvent (such as THF) regarding any potential for flammability. This charging with polar organic solvent may be done by aspirating the solvent into the vessel and is preferrably done without letting air into the vessel as a safety precaution.

After addition of the polar solvent has been completed, the temperature of the reaction vessel is lowered (such as by using a cooling jacket) to a temperature in the range of (-5) - (20) degrees C. and preferably 0 - 10 degrees (for example, 0 degrees). A nitrogen bleed is then resumed to the vessel to create a positive pressure of the inert gas. Sufficient caustic is added via the funnel described above to maintain the pH in the reaction vessel at a value in the range of 9 - 13, preferably at a pH of 9 - 10 initially.

During the time the pH is being adjusted, a chloride of Formula III (for example, lauroyl chloride) is being added to the reaction vessel in an amount such that:

(a) it does not exceed 95% of the molar equivalent amount of the compound of Formula II added initially;
(b) the pH value is maintained in the range of 9 - 13; and
(c) the temperature of the reaction mixture is maintained in the range of 0 - 10 degrees C. (for example, in the range of 2 - 10 degrees).

It is preferred that the pH be maintained at a value of about 10 at the outset of the addition of the chloride of Formula III and that the pH be allowed to gradually rise to a value of 12.5 - 13 during the final stages of the addition of the chloride.

In order to optimize the yield and product quality the temperature of the reaction should be maintained in the range of 0 - 10 degrees C; it is preferred that a temperature at the lower end of this range be used, such as 2 - 5 degrees C. The temperature can be controlled by reactor heat transfer mechanisms, such as with the use of cooling coils or a cooling jacket, and regulating the addition rate of the compound of Formula III. After the charge has been completed, the contents of the reaction vessel are held at a temperature of 2 - 8 degrees for an additional period of time in the range of 0.5 - 2 hours (for example, 30 minutes) at a pH greater than or equal to 11, such as a pH of 11 - 12.

Next the reaction mixture is drowned into a mineral acid. The acid or some dilution of the acid may be added directly to the reaction mixture with later addition of dilution water or the reaction mixture can be poured into an acid solution, provided that in each case sufficient dilution water is included which will equal 5 - 10 times the volume of the original reaction mixture. Suitable acids include HCl, $H_2SO_4$ and $H_3PO_4$ and especially HCl solution.

One example of a method for accomplishing this drowning step is as follows. In the drowning vessel (now empty) is placed a volume of water which is approximately five times the volume of the reaction mixture with sufficient HCl to obtain a pH value of 1- 2. If the volume of the vessel does not permit this, the process may have to be accomplished in portions. The reaction mixture is slowly transferred into the drowning vessel with vigorous mixing. This addition is accomplished at a rate such that the temperature of the mixture remains in the range of 10 - 70 degrees C. and preferably in the range of 15 - 20 degrees. HCl is added as needed to keep the pH of the contents of the drowning vessel below 3. Depending on the relative volumes of the reaction and drowning vessels available, it may be necessary to drown the reaction mixture in portions using techniques known to those skilled in the art with periodic filtration as needed. The particle size of the product depends on the temperature, amount of water used, addition rate of the acid chloride, stirring rate and drowning rate. It is preferred that these parameters be adjusted according to the vessel size and reaction conditions so that optimal particle size is obtained for the product. This means that the particle size is large enough to filter well and is preferably uniform in size. If the particle size is too small the filter may become blocked in the next step.

After this step has been completed, complete conversion of the product to the diacid form is effected. Additional acid solution (such as a solution made with HCl) is added to the reaction mixture (all or portions at a time if that is necessary) until the pH of the mixture is below 3, such as in the range of 1.0 - 1.5. Preferably, the mixture is then heated to a temperature of 60 - 65 degrees and held at this temperature for a period of 0.25 - 1 hour (for example, 0.5 hour). The mixture is then cooled to ambient temperature, such as 25 degrees C (such as with the use of brine, ice water or ethylene glycol in the cooling jacket of the reaction vessel). The mixture is held at ambient temperature until it is filtered to recover the product. It is preferable that the product be filtered promptly to optimize the quality of the product recovered. The filtrate may be disposed of in any acceptable manner.

The press cake from the filtration process is washed with water until the electrical conductivity of the outlet water is approximately the same as the inlet water. This is conveniently done using tap water initially and then switching to deionized water for the final washes. The press cake is squeezed and blown, preferably with nitrogen, to remove

more water. The product is dried (for example, on a tray dryer) as required.

It is to be noted that the contact of locally high concentrations of acid chloride and the base (for example, lauroyl chloride and sodium hydroxide) should be carefully avoided by proper location of reagent inlets, configuration of baffles, and design and speed of the stirrer.

EXAMPLES

The following Examples are illustrative of the invention but should not be construed as limitations thereon. Unless otherwise indicated, all chemical symbols, abbreviations and nomenclature have their usual and customary meanings. Percents are in weight percents, temperatures are measured in degrees Centigrade. Unless otherwise specified, the caustic used in the Examples is a 50 percent solution of sodium hydroxide.

EXAMPLE 1

A reaction vessel of 11,370 liter capacity is charged with monosodium glutamate (1045.5 kg) dissolved in 2464 liters of water. The reaction vessel is purged 3 times with nitrogen at a pressure of 206.8 kPa (30 psi). Full vacuum is applied to the vessel and suction is used to charge 2462 liters of isopropanol. Care is taken to ensure that no air is allowed to be aspirated during this step. The vacuum is then broken and the vessel is charged with nitrogen to create a nitrogen atmosphere. Using cooling brine in a jacket around the vessel, the temperature of the contents of the reaction vessel is then lowered to a temperature of 0 - 5 degrees C. A nitrogen bleed is begun to the vessel. Caustic (46.6 liters of 50 percent solution) is added to the reaction vessel to adjust the pH to a value of 10. Lauroyl chloride (1090 kg) is added to the reaction vessel at a rate of 4 - 5 kg per minute. The pH of the contents of the reaction vessel is maintained in a range of 9 - 13 at all times using a solution of 50 percent caustic. It is preferred that the pH be maintained at a value of 10 at the outset and that the pH be allowed to gradually rise to a final value of 12 - 13 during the addition of lauroyl chloride. The temperature of the reaction is maintained between 2 - 10 degrees C. The temperature is controlled by a reactor heat transfer mechanism and by regulating the addition rate for the lauroyl chloride. After the charge is completed, the contents of the reaction vessel are held at a temperature of 2 - 8 degrees C for an additional 30 minutes at a pH of ≥ 11 (for example, 11 - 12). A separate reaction vessel is prepared (Vessel B) containing Hcl solution. The original reaction vessel (Vessel A) is pressurized with 137.9 - 206.8 kPa (20 - 30 psi gauge (psig)) nitrogen to transfer its contents to Vessel B and excess nitrogen is vented to the scrubber. Additional water is added to Vessel A in an amount sufficient to remove the product from Vessel A and rinse the contents of Vessel A into Vessel B. The mixture of the contents of Vessel A and the additional water is added to Vessel B in such a manner that the pH is kept below 3.

Alternatively, this last procedure can be done in 1/4 increments because of the size of the vessels used. Filtration is done between each 1/4 of the material that is processed. In one particular case a total of 7600 gallons ( 28,769 liters) of water and 3176 pounds (1,185.42 kg.) of 31 percent Hcl were used in quater portions.

The reaction mixture which has just been mixed with the HCl solution is adjusted to a pH of 1.0 - 1.5 with additional HCl. The drowned mixture in Vessel B is heated to a temperature of 60 - 65 degrees C and held at this temperature for 30 minutes. The mixture is then cooled to ambient temperature (about 25 degrees C.) using brine in the cooling jacket of the reaction vessel. The mixture is held at this temperature for 90 minutes. The mixture is then filtered, saving the solid and disposing of the filtrate properly. The solid on the filter (also called filter cake or press cake) is washed first with tap water and then with deionized water until the conductivity of the outlet water is approximately the same as that of the inlet water. The press cake is squeezed and blown with nitrogen to remove more water. The product is dried on a tray dryer. Typically the experimental yield using this procedure is 1539 - 1572 kg (94 - 96 percent) with a purity of about 96 percent, a melting point of 104 - 106 degrees C.

**Claims**

**1.** A process for making N-acyl-aminodiacids of Formula I:

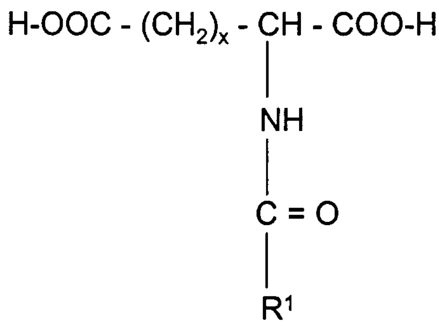

Formula I

wherein:

$R^1$ is selected from the group consisting of $C_1$-$C_{20}$ alkenyl, and $C_2$-$C_{20}$ alkynyl, wherein the alkenyl and alkynyl groups may contain up to three unsaturations and wherein all of the groups may optionally contain branching; and
x=0-10;

wherein said process comprises

(a) a reacting an excess of a compound of Formula II:

$$M^1 - OOC - CH - (NH_2) - (CH_2)_x - COOM^2 \qquad \text{Formula II}$$

wherein:
$M^1$ and $M^2$ are each independently selected from the group consisting of hydrogen, sodium, potassium and ammonium with a chloride of Formula III:

$$R^1 - C(O)Cl \qquad \text{Formula III}$$

in the presence of a water/polar organic solvent mixture, said solvent selected from the group of methanol, ethanol, n-propanol, isopropanol, n-butanol, isobutanol, tert.-butanol and tetrahydrofuran, with a sufficient amount of base selected from the group of NaOH, KOH, $Na_2CO_3$ and $K_2CO_3$ to form a reaction mixture from which a disalt form of the compound of Formula I will be obtained; provided that the compound of Formula III is added in an amount such that:

(a) it does not exceed 95 % of the molar equivalent amount of the compound of Formula II added initially;
(b) the pH value of the reaction mixture is maintained in the range of 9-13; and
(c) the temperature of the reaction mixture is maintained in the range of 0-10 degrees C; and

(b) combining the disalt of Formula (I) with a mineral acid solution simultaneously or sequentially in 5 to 10 times the volume of original reaction mixture of dilution water to form the compound of Formula I.

**2.** A process as claimed in Claim 1 wherein x = 1-4.

**3.** A process as claimed in Claim 1 wherein $R^1 = C_{11}$ and x = 2.

**4.** A process as claimed in Claim 1 wherein the compound of Formula II is monosodium glutamate.

**5.** A process as claimed in claim 1 wherein the polar organic solvent is selected from the group consisting of ethanol, n-propanol and isopropanol.

**Patentansprüche**

**1.** Verfahren zur Herstellung von N-Acylamidodicarbonsäuren der Formel I:

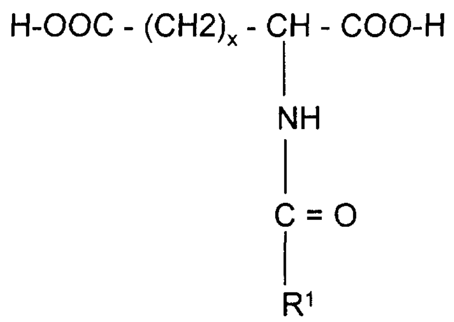

Formel I

in der:

$R^1$ aus der Gruppe $C_1$-$C_{20}$-Alkenyl und $C_2$-$C_{20}$-Alkinyl ausgewählt ist, wobei die Alkenyl- und Alkinylgruppen bis zu dreifach ungesättigt sein können und wobei sämtliche Gruppen gegebenenfalls verzweigt sein können; und

x = 0 - 10;

wobei man

(a) einen Überschuss einer Verbindung der Formel II:

$$M^1 - OOC - CH - (NH_2) - (CH_2)_x - COOM^2 \qquad \text{Formel II}$$

in der:
$M^1$ und $M^2$ jeweils unabhängig voneinander aus der Gruppe Wasserstoff, Natrium, Kalium und Ammonium ausgewählt sind, umsetzt mit einem Chlorid der Formel III:

$$R^1\text{-C(O)Cl} \qquad \text{Formel III}$$

in Gegenwart einer Mischung aus Wasser und polarem organischem Lösungsmittel aus der Gruppe Methanol, Ethanol, n-Propanol, Isopropanol, n-Butanol, Isobutanol, tert.-Butanol und Tetrahydrofuran, mit einer ausreichenden Menge einer Base aus der Gruppe NaOH, KOH, $Na_2CO_3$ und $K_2CO_3$ um ein Reaktionsgemisch zu bilden, aus dem eine Disalzform der Verbindung der Formel I erhalten werden soll, mit der Maßgabe dass die Verbindung der Formel III in einer solchen Menge zugegeben wird, dass

(a) sie 95 % der molar äquivalenten Menge der ursprünglich zugegebenen Menge der Verbindung der Formel II nicht übersteigt,

(b) der pH-Wert des Reaktionsgemisches im Bereich von 9 - 13 gehalten wird, und

(c) die Temperatur des Reaktionsgemisches im Bereich von 0 - 10°C gehalten wird, und

(b) das Disalz der Formel (I) mit einer Mineralsäurelösung gleichzeitig oder nacheinander in der 5- bis 10-fachen Menge von Verdünnungswasser, bezogen auf das Volumen des Ausgangsreaktionsgemisches, unter Bildung der Verbindung der Formel I vereinigt.

**2.** Verfahren nach Anspruch 1, wobei x = 1 - 4.

3. Verfahren nach Anspruch 1, wobei $R^1 = C_{11}$ und x = 2.

4. Verfahren nach Anspruch 1, wobei die Verbindung der Formel II Mononatriumglutamat ist.

5. Verfahren nach Anspruch 1, wobei das polare organische Lösungsmittel aus der Gruppe Ethanol, n-Propanol und Isopropanol ausgewählt ist.

## Revendications

1. Procédé pour la préparation des acides dicarboxylique de Formule I:

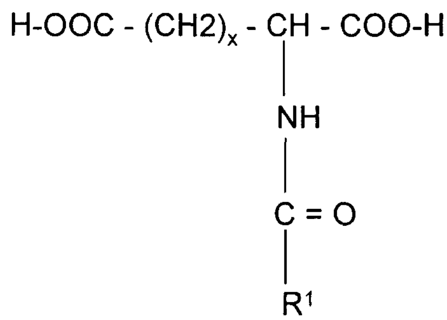

Formule I

dans laquelle:

$R^1$ est choisi dans la classe formée par des groupes alkyle en $C_1$-$C_{20}$, alcényle en $C_2$-$C_{20}$ et alcynyle en $C_2$-$C_{20}$, Les groupes alcényle et alcynyle pouvant contenir jusqu'à trois insaturations et tous les groupes pouvant éventuellement être ramifiés; et

$$x = 0 \text{ à } 10;$$

ledit procédé consistant à
(a) faire réagir un excès d'un composé de Formule II:

$$M^1 - OOC - CH - (NH_2) - (CH_2)_x - COOM^2 \qquad \text{Formule II}$$

dans laquelle $M^1$ et $M^2$ sont chacun indépendamment choisis dans la classe formée par l'hydrogène, le sodium, le potassium et l'ammonium, avec un chlorure de Formule III:

$$R^1\text{-}C(O)Cl \qquad \text{Formule III}$$

es présence d'un mélange d'eau et d'un solvant organique polaire, ledit solvant étant choisi dans la classe formée par le méthanol, l'éthanol, le n-propanol, l'isopropanol, le n-butanol, l'isobutanol, le tert-butanol et le tétra-hydrofuranne, avec une quantité suffisante d'une base choisie dans la classe formée par NaOH, KOH, $Na_2CO_3$ et $K_2CO_3$ pour former un mélange réactionnel à partir duquel on obtient le composé de Formule I sous forme d'un di-sel; étant entendu que le composé de Formule III est ajouté en une quantité telle que:

(a) elle ne dépasse pas 95 % de la quantité molaire équivalente du composé de Formule II ajouté initialement;
(b) le pH du mélange réactionnel soit maintenu dans l'intervalle de 9 à 13; et
(c) la température du mélange réactionnel soit maintenue dans l'intervalle de 0 à 10 degrés C; et

(b) combiner le di-sel de Formule (I) avec une solution d'acide minéral, simultanément ou successivement, dans un volume d'eau de dilution de 5 à 10 fois le volume du mélange réactionnel initial, pour forme le composé de Formule I.

**2.** Procédé selon la revendication 1, dans lequel x = 1 à 4.

**3.** Procédé selon la revendication 1, dans lequel $R^1 = C_{11}$ et x = 2.

**4.** Procédé selon la revendication 1, dans lequel le composé de Formula II est le glutamate monosodique.

**5.** Procédé selon la revendication 1, dans lequel le solvant organique polaire est choisi dans la classe formée par l'éthanol, le n-propanol et l'isopropanol.